(19) Europäisches Patentamt
European Patent Office
Office européen des brevets

(11) Veröffentlichungsnummer: **0 311 943**
**A1**

(12) # EUROPÄISCHE PATENTANMELDUNG

(21) Anmeldenummer: **88116748.0**

(51) Int. Cl.⁴: **A61L 15/00 , C08G 63/66**

(22) Anmeldetag: **10.10.88**

(30) Priorität: **12.10.87 NL 8702422**

(43) Veröffentlichungstag der Anmeldung:
**19.04.89 Patentblatt 89/16**

(84) Benannte Vertragsstaaten:
**AT BE CH DE ES FR GB GR IT LI LU NL SE**

(71) Anmelder: **Akzo N.V.**
**Postbus 9300 Velperweg 76**
**NL-6800 SB Arnhem(NL)**

(72) Erfinder: **Vrouenraets, Cornelius Martinus Franciscus, Dr.**
**Eduard van Beinumlaan 121**
**NL-6952 CL Dieren(NL)**
Erfinder: **Herberts, Hans Reinirus**
**Braambergseweg 18**
**NL-6996 AL Drempt(NL)**

(74) Vertreter: **Fett, Günter**
**Akzo Patente GmbH Kasinostrasse 19 - 23**
**D-5600 Wuppertal 1(DE)**

(54) **Absorptionsfähiges Hygieneprodukt.**

(57) Absorptionsfähiges Hygieneprodukt wie beispielsweise Windeln, Unterlagen, Damenbinden, bestehend aus einer Copolyetheresterfolie mit einer Wasserdurchlässigkeit von maximal 0,02 g/cm²/Std.23 °C/0,07 bar und einer Wasserdampfdurchlässigkeit von mindestens 500 g/m²/Tag/23 °C/100 -50% rel. Luftfeuchte, einer flüssigkeitsdurchlässigen Schicht und einer flüssigkeitsaufnehmenden Sauglage, wobei der Copolyetherester der Folie einen Gewichtsanteil von 5 - 55 Gew.-% an langkettigen Glycolen mit einem Molekulargewicht von 800 - 6000 mit einem Atomverhältnis Kohlenstoff zu Sauerstoff von 2,0 bis 3,4 enthält und die Schnitzel des Copolyetheresters bei 23 °C in Wasser eine Wasseraufnahme von höchstens 15 Gew.-% aufweisen.

EP 0 311 943 A1

## Absorptionsfähiges Hygieneprodukt

Die Erfindung betrifft ein absorptionsfähiges Hygieneprodukt, wie beispielsweise Windeln, Unterlagen, Damenbinden, bestehend aus einer Folie mit geringer Flüssigkeitsdurchlässigkeit und einer Wasserdampfdurchlässigkeit von mindestens 500 g/m$^2$/Tag/ 23$^\circ$C/100-50 % relative Luftfeuchtigkeit/Windgeschwindigkeit 0,3 m/s, einer flüssigkeitsdurchlässigen Schicht und einer flüssigkeitsaufnehmenden Sauglage, wobei die Folie aus einem Copolyetherester hergestellt ist, der aus einer Vielzahl sich wiederholender intralinearer Estergruppen mit einem langen Kettenmolekül und Estergruppen mit einem kurzen Kettenmolekül besteht, die durch Esterbindungen auf statistische Weise so miteinander verbunden sind, daß der Anfang und das Ende jeweils aneinander angrenzen, wobei die Estergruppen mit langkettigen Estereinheiten der folgenden Formel entsprechen:

$$-OGO\overset{\overset{O}{\|}}{C}R\overset{\overset{O}{\|}}{C}-$$

und wobei die Estergruppen mit einem kurzkettigen Estereinheiten der folgenden Formel entsprechen:

$$-ODO\overset{\overset{O}{\|}}{C}R\overset{\overset{O}{\|}}{C}-$$

wobei G einen zweiwertigen Rest darstellt, die nach Entfernung von endständigen Hydroxylgruppen von mindestens einem langkettigen Glycol, einem Molekulargewicht von mehr als 800 und einem mittleren Atomverhältnis von Kohlenstoff zu Sauerstoff von 2,0 übrigbleibt, wobei R einen zweiwertigen Rest darstellt, der nach Entfernung von Carboxylgruppen von mindestens einer Carbonsäure mit einem Molekulargewicht von weniger als 300 übrigbleibt, und D einen zweiwertigen Rest darstellt, der nach Entfernung von Hydroxylgruppen von mindestens einem Diol mit einem Molekulargewicht von weniger als 250 übrigbleibt, wobei die verwendete Dicarbonsäure zu mindestens 75 Mol% aus Terephthalsäure oder deren esterbildenden Äquivalenten besteht und das Diol mit einem niedrigen Molekulargewicht zu mindestens 75 Mol% aus 1,4-Butandiol oder dessen esterbildenden Äquivalenten besteht, die Summe der Molprozente der Dicarbonsäure, die keine Terephthalsäure oder dessen esterbildendes Äquivalent ist, und des Diols mit einem niedrigen Molekulargewicht, das kein 1,4-Butandiol oder dessen esterbildendes Äquivalent ist, höchstens 25 beträgt.

Aus der japanischen Offenlegungsschrift 51/111 290 ist ein hygienischer Artikel mit einer entsprechenden Folie bereits bekannt, wobei der Copolyetherester aus langkettigen und kurzkettigen Estereinheiten aufgebaut ist und die kurzkettigen Estereinheiten 30 - 40 Gew.-% des Copolyetheresters ausmachen und die langkettigen Einheiten Estereinheiten von Terephthalsäure und Polyäthylenglykol mit einem Molekulargewicht von 800 - 3.000 sind.

In der europäischen Patentanmeldung 192 965 wird ein ähnliches Produkt beschrieben, bei dem als nahezu flüssigkeitsundurchlässige, jedoch wasserdampfdurchlässige Folie eine monoaxial oder biaxial verstreckte poröse Folie aus einem Polyolefin dient mit einer Dicke von maximal 60 µm, einer Porengröße zwischen 0,05 und 5 µm, einem Porenvolumen von mindestens 0,1 cm$^3$ pro cm$^3$folie, einer Wasserdampfdurchlässigkeit von mindestens 500 g/m$^2$/Tag und einer Biegefestigkeit (in mm), die höchstens der 0,193-fachen Foliendicke (in µm) + 35 entspricht.

Obwohl die dort beschriebenen Produkte eine Verbesserung gegenüber den bekannten wasserdampfundurchlässigen Produkten darstellen, besteht ein großer Bedarf an absorptionsfähigen Hygieneprodukten mit verbesserten Eigenschaften, z.B. einer höheren Wasserdampfdurchlässigkeit bei gleicher oder höherer Wasserdichtheit.

Durch die Erfindung wurde nunmehr ein absorptionsfähiges Hygieneprodukt gefunden, das die genannten verbesserten Eigenschaften besitzt, die sich zudem auf einfache Weise realisieren lassen.

Die Erfindung ist dadurch gekennzeichnet, daß das absorptionsfähige Hygieneprodukt der obengenannten bekannten Art eine Folie aus Copolyetherester enthält, die zu 45 - 95 Gew.% aus Estergruppen mit kurzkettigen Estereinheiten besteht, die Flüssigkeitsdurchlässigkeit von maximal 0,02 g/cm²/Std/23 °C/0,07 bar
und im Copolyetherester der Gewichtsanteil der langkettigen Estereinheiten 5 - 55% beträgt und das mittlere Atomverhältnis Kohlenstoff/Sauerstoff des langkettigen Glycols zwischen 2,0 und 3,7 liegt mit der Maßgabe, daß, wenn das mittlere Atomverhältnis Kohlenstoff zu Sauerstoff der langkettigen Glycole zwischen 2,0 und 2,4 liegt, der Gewichtsanteil der langkettigen Estereinheiten 5 - 35% beträgt und die Copolyetherester-Folie durch Flach- und/oder Blasextrusion aus Polymerschnitzeln, die in Wasser von 23 °C eine Wasseraufnahme von höchstens 15 Gew.%, bezogen auf das Trockengewicht der Schnitzel, aufweisen, hergestellt wurden.

Es sei hier darauf hingewiesen, daß in der US-Patentschrift 4 493 870 ein Teil der obengenannten Copolyetherester für die Herstellung von Folien für den Einsatz in wasserdichter Regenkleidung und Zelten empfohlen wird. Die Mehrzahl der in den Beispielen beschriebenen Copolyetheresterfolien ist jedoch für die Herstellung von absorptionsfähigen Hygieneprodukten ungeeignet, da die Folien unter Bedingungen, wie sie beim Einsatz von Hygieneprodukten auftreten können, zu stark wasserdurchlässig sind. Hinzu kommt, daß bei Folien für den Einsatz in den letztgenannten Produkten sehr hohe Anforderungen an die Geschmeidigkeit des Materials gestellt werden. Schließlich müssen die Herstellungskosten dieser Folien in einer annehmbaren Größenordnung liegen.

Neben den Copolyetheresterfolien mit den angegebenen Eigenschaften enthalten die erfindungsgemäßen Hygieneprodukte eine in hohem Maße wasserdurchlässige Schicht. Diese Schicht besteht meistens aus einem Gewebe oder einem Vlies aus einem vorzugsweise hydrophoben Material. Dabei ist insbesondere an ein Vliesprodukt zu denken aus einem Polyolefin, z.B Polyethylen und insbesondere Polypropen oder dessen Copolymeren. Daneben kommen auch - vorzugsweise Vlies- Produkte aus anderen hydrophoben Materialien, z.B. Polyester, in Frage.

Für die Herstellung von Copolyetherestern für den Einsatz in den erfingungsgemäßen absorptionsfähigen Hygieneprodukten kann auf die britischen Patentschriften 682 866, 1 403 210 und 1 404 340 verwiesen werden.

Vorzugsweise werden für erfindungsgemäße absorptionsfähige Hygieneprodukte Copolyetherester eingesetzt, die 60 - 90 Gew.-% kurzkettige Estereinheiten enthalten.

Als Glycol für die Bildung langkettiger Estereinheiten mit einem mittleren Atomverhältnis Kohlenstoff/Sauerstoff von 2,0 - 3,7 wird vorzugsweise ein Polyalkylenoxidglycol mit einem Molekulargewicht zwischen 1000 und 5000 eingesetzt.

Ferner wird im Rahmen der vorliegenden Erfindung Copolyetherestern der Vorzug gegeben, deren Estergruppen mit kurzkettigen Estereinheiten ganz oder hauptsächlich aus Polybutylen-Terephthalat-Gruppen bestehen. Folien aus diesen Copolyetherestern sind leicht herzustellen. Zudem weisen Folien aus diesem Material im allgemeinen bei der zur Diskussion stehenden Anwendung bessere technische Eigenschaften auf als Folien aus Copolyetherestern, bei denen z.B. 25 % der Terephthalsäure durch eine andere Dicarbonsäure ersetzt wurde. Bei speziellen Anwendungen kann sich der Ersatz eines geringen Anteils des 1,4-Butandiols durch ein anderes Diol und/oder der Ersatz der Terephthalsäure durch eine andere Dicarbonsäure mit einem geringen Molekulargewicht als vorteilhaft erweisen. Zu den Diolen mit niedrigem Molekulargewicht (außer 1,4-Butandiol), die zu Estergruppen mit einem kurzen Kettenmolekül umgesetzt werden, gehören acyklische, alicyklische und aromatische Dihydroxyverbindungen. Zu bevorzugen sind Diole mit 2 - 15 Kohlenstoffatomen, z.B. Ethylen-, Propylen-, Isobutylen-, Pentamethylen-, 2,2-Dimethyltrimethylen-, Hexamethylen- und Dekamethylenglycol, Dihydroxycyclohexan, Cyclohexandimethanol, Resorcinol, Hydrochinon und 1,5-Dihydroxynaphthalen. Insbesondere sind aliphatische Diole mit 2 - 8 Kohlenstoffatomen zu bevorzugen.
Zu den geeigneten Bisphenolen gehören Bis(p-hydroxy)biphenyl, Bis(p-hydroxyphenyl)methan und Bis(p-hydroxyphenyl)propan. Entsprechende esterbildende Derivate von Diolen sind ebenfalls geeignet (z.B. kann man anstelle von Ethylenglykol Epoxyethan oder Ethylencarbonat verwenden). Auf derartige entsprechende esterbildende Derivate bezieht sich der Ausdruck "Diole mit niedrigem Molekulargewicht", wobei sich jedoch die Forderung bezüglich des Molekulargewichts auf das Diol selbst und nicht auf dessen Derivate bezieht.

Zu den Dicarbonsäuren (abgesehen von Terephthalsäure), die mit den genannten Glycolen mit einem langen Kettenmolekül und mit Diolen mit niedrigem Molekulargewicht zu Copolyestern umgesetzt wurden, gehören aliphatische, cycloaliphatische und aromatische Dicarbonsäuren mit einem Molekulargewicht von maximal 300. Unter dem Begriff "Dicarbonsäure" werden hier auch Äquivalente von Dicarbonsäuren mit zwei Carboxyl-Funktionsgruppen verstanden, die sich bei der Umsetzung mit Glycolen und Diolen zu Copolyestern nahezu gleich verhalten wie die Dicarbonsäuren. Zu diesen Äquivalenten gehören Ester und

esterbildende Derivate, z.B. die Säurehalogenide und Anhydride. Die Forderungen bezüglich des Molekulargewichts beziehen sich auf die Säure und nicht auf die äquivalenten Ester oder deren esterbildende Derivate. Die Dicarbonsäuren können beliebige substituierte Gruppen oder Kombinationen umfassen, die die Copolyesterbildung und die Anwendung des Polymers in den elastomeren Produkten gemäß der vorliegenden Erfindung nicht stören. Der Ausdruck "aliphatische Dicarbonsäuren" bezieht sich hier auf Carbonsäuren mit zwei Carboxylgruppen, die jeweils an ein gesättigtes Kohlenstoffatom gebunden sind. Aliphatische oder cycloaliphatische Säuren mit konjugierten ungesättigten Bindungen können wegen der Homopolymerisation häufig nicht verwendet werden. Einige ungesättigte Säuren, z.B. Maleinsäure, sind jedoch geeignet. Der Ausdruck "aromatische Dicarbonsäuren" bedeutet hier Dicarbonsäuren mit zwei Carboxylgruppen, die an je ein Kohlenstoffatom eines Benzolrings oder eines mehrkernigen aromatischen Ringsystems gebunden sind. Es ist nicht erforderlich, daß beide Carboxylfunktionsgruppen an den gleichen aromatischen Ring gebunden sind. Sind mehrere Ringe vorhanden, so können sie durch aliphatische oder aromatische oder andere zweiwertige Gruppen, z.B. -O- oder $-SO_2-$ verbunden sein.
Vorzugsweise werden Cyclohexandicarbonsäuren und Adipinsäure eingesetzt.

Zu den geeigneten aromatischen Dicarbonsäuren gehören Phthalsäure und Isophthalsäure, Bisbenzoesäure, substituierte Dicarboxylverbindungen mit zwei Benzolkernen, z.B. Bis(p-carboxphenyl)methan, P-oxy-(p-carboxyphenyl)benzoesäure, Ethylen-bis(p-oxybenzoesäure), 1,5-Naphthalindicarbonsäure, 2,6-Naphthalindicarbonsäure, 2,7-Naphthalindicarbonsäure, Phenantrendicarbonsäure, Antrazindicarbonsäure, 4,4′-Sulphonylbibenzoesäure und deren mit Alkylgruppen mit 1 - 12 Kohlenstoffatomen und am Ring substituierten Derivaten, z.B. Halogen-, Alkoxy- und Aryl- derivaten. Hydroxysäuren, z.B. p-(β-Hydroxyethoxa)-Benzoesäure lassen sich ebenfalls verwenden unter der Voraussetzung, daß auch eine aromatische Dicarbonsäure vorhanden ist.

Bei der Herstellung der Copolyester werden bevorzugt aromatische Dicarbonsäuren verwendet, insbesondere Säuren mit 8-16 Kohlenstoffatomen, vornehmlich Phenylendicarbonsäuren, d.h. Phthalsäure und Isophthalsäure.

Erfindungsgemäß muß das langkettige Glycol ein mittleres Atomverhältnis Kohlenstoff/Sauerstoff zwischen 2,0 und 3,7 aufweisen. Dabein kann es sich um die Anwendung von lediglich einem Polyalkylenoxidglycol mit einem Kohlenstoff/ Sauerstoff-Verhältnis zwischen 2,0 und 3,7 handeln, oder eines Gemisches aus einem Polyethylenoxidglycol mit einem Kohlenstoff/Sauerstoff-Verhältnis von 2,0 mit einem Polyalkylenoxidglycol mit einem höheren Kohlenstoff/Sauerstoff-Verhältnis, wovon z.B. die Enden mit Polyethylenoxidgruppen blockiert sind. Geeignet ist auch ein Polyalkylenoxidglycol, das man durch Mischpolymerisation von Ethylenoxid und einem zweiten Epoxyalkan erhält. Der Anteil dieses zweiten Monomers am Polyalkylenoxidglycol beträgt vorzugsweise weniger als 80 Mol% oder, wenn das mittlere Atomverhältnis Kohlenstoff zu Sauerstoff zwischen 2,0 und 2,4 liegt, weniger als 20 Mol%. Geeignete Beispiele von zweiten Monomeren sind 1,2-und 1,3-Epoxypropan, 1,2-Epoxybutan und Tetrahydrofuran.

Das Kohlenstoff-Sauerstoff-Verhältnis von 2,0 bei den langkettigen Glycolen führt im Rahmen der Erfindung nur dann zu geeigneten Copolyestern, wenn der Anteil an kurzkettigen Estereinheiten hoch ist, da sonst die Wasseraufnahme der Polymerschnitzel über 15 Gew.-% ansteigt,
was aber zugleich zu einer Verschlechterung der Wasserdichtheit führt. Verringert man den Anteil an kurzkettigen Estereinheiten, so muß im Polyalkylenglykol das Kohlenstoff-Sauerstoff-Verhältnis bis auf 3,7 durch Zusatz anderer Polyethermonomere angehoben werden, um geringe Wasseraufnahme der Polymerschnitzel und Wasserdichtigkeit der Copolyesterfolie zu gewährleisten.

Die hier beschriebenen Polymere lassen sich auf einfache Weise mit einer konventionellen Umesterung herstellen. Bei einem bevorzugten Verfahren wird der Terephthalsäure-Dimethylester mit einem langkettigen Glycol und einem molaren Überschuß an Butandiol unter Verwendung eines Katalysators auf 150 - 260° C erhitzt, wonach das bei der Umesterung entstandene Methanol abdestilliert wird. Dann wird weiter erhitzt, bis die Methanol-Entwicklung beendet ist. Die Umesterung vollzieht sich innerhalb von wenigen Minuten bis zu einigen Stunden, wobei sich die Dauer nach der Temperatur, der Art des Katalysators und der Höhe des Diol-Überschusses richtet. Mit diesem Verfahren erhält man ein Vorpolymer mit einem niedrigen Molekulargewicht, das sich nach dem nachfolgend beschriebenen Verfahren zu einem Copolyester mit einem hohen Molekulargewicht umsetzen läßt. Derartige Vorpolymere lassen sich auch mit einer Reihe anderer Veresterungs- oder Umesterungsverfahren herstellen; das langkettige Glycol kann z.B. mit einem Homopolymer oder einem Copolymer eines Esters mit kurzen Kettenmolekülen und einem hohen oder niedrigen Molekulargewicht unter Verwendung eines Katalysators umgesetzt werden, bis eine willkürliche Verteilung entsteht. Homopolymere oder Copolymere von Estern mit kurzkettigen Estereinheiten können durch Umesterung aus Dimethylestern und Diolen mit niedrigem Molekülgewicht, wie hier beschrieben, oder aus freien Säuren mit Diolacetaten hergestellt werden. Das Estercopolymer mit kurzkettigen Estereinheiten läßt sich auch durch direkte Veresterung aus geeigneten Säuren, Anhydriden oder Säurechloriden, z.B. mit

Diolen oder mit anderen Verfahren, z.B. durch Umsetzung der Säuren mit cyclischen Ethern oder Carbonaten herstellen. Das Vorpolymer erhält man auch bei Durchführung dieses Verfahrens in Anwesenheit eines langkettigen Glycols.

Danach wird das erhaltene Polymer durch Abdestillierung des Überschusses an Diol mit kurzen Kettenmolekülen zu einem Produkt mit hohem Molekulargewicht umgesetzt. Dieses Verfahren ist als "Polykondensation" bekannt. Bei dieser Destillation findet noch immer eine Umesterung statt, um das Molekulargewicht zu erhöhen und um eine willkürliche Anordnung der Copolyestergruppen zu erhalten. Das beste Ergebnis erzielt man normalerweise, wenn diese Enddestillation bzw. Polykondensation bei einem Druck von höchstens 130 Pa und bei 240 - 260° C höchstens 2 Stunden lang in Anwesenheit von Antioxidationsmitteln, z.B. symm. Di-β-naphtyl-p-phenylendiamin und 1,3,5-Trimethyl-2,4,6-tris[3,5-ditert.butyl-4-hydroxybenzyl]-benzol durchgeführt wird. Die Polymerisa tionstechnik mit der größten praktischen Bedeutung ist die Umesterung, mit deren Hilfe die Polymerisationsreaktion zu Ende geführt wird. Um zu vermeiden, daß die Produkte zu lange hohen Temperaturen ausgesetzt werden mit der Folge eines möglicherweise irreversiblen Abbaus durch die Hitzeeinwirkung, ist es zweckmäßig, bei der Umesterung einen Katalysator zu verwenden. Hierfür ist eine große Anzahl von Katalysatoren geeignet; bevorzugt weden jedoch organische Titanate eingesetzt, z.B. Tetrabutyl-Titanat, da man allein oder zusammen mit Magnesium- oder Calciumacetat verwenden kann. Komplexe Titanate, z.B. $Mg[HTi(OR)_6]_2$, die man aus Alkali- oder Erdalkalimetalalkoxiden und Titanatestern herstellt, sind ebenfalls gut geeignet. Anorganische Titanate, z.B. Lanthantitanat, Calciumacetat/Antimontri oxyd-Gemische und Lithium- und Magnesiumalkoxide sind Beispiele weiterer Katalysatoren, die sich ebenfalls verwenden lassen.

Erfindungsgemäß verwendet man als langkettiges Glykol stets ein Glycol mit einem Molekulargewicht zwischen 800 und 6000.

Bei Einsatz eines Glycols mit einem Molekulargewicht von <800 muüte eine unannehmbar hohe Menge des Glycols von den Copolyetherestern aufgenommen werden. Eine hieraus hergestellte Folie mit einer Dicke von z.B. 35 µm und einer Wasserdampfdurchlässigkeit von mindestens 500 g/m²/Tag/23° C/100-50 % rel. Luftfeuchtigkeit bei einer Windgeschwindigkeit von 0,3 m/s hat sich aufgrund ihrer technischen Eigenschaften für den Einsatz in absorptionsfähigen Hygieneprodukten als weniger gut geeignet erwiesen. Auch die Herstellung von absorptionsfähigen Hygieneprodukten mit Copolyetheresterfolien aus Copolyetherestern, die mit einem Glycol mit einem Molekulargewicht von > 6000 hergestellten sind, führt zu unbefriedigenden Ergebnissen, da die technischen Eigenschaften der Copolyetheresterfolien teilweise, z.B. im Hinblick auf die Festigkeit, den Anforderungen nicht genügen.

Sowohl die Wasserdurchlässigkeit als auch die Wasserdampfdurchlässigkeit der in den vorliegenden Produkten einzusetzenden Copolyetheresterfolien sind nicht nur von deren Zusammensetzung, sondern auch von ihrer Dicke abhängig. Bei jeder gewählten Foliendicke darf die Wasserdurchlässigkeit höchstens 0,02 g/cm²/Std/23° C/0,07 bar betragen, während die Wasserdampfdurchlässigkeit bei mindestens 500 g/m²/Tag/23° C/100-50 % rel. Luftfeuchtigkeit/Windgeschwindigkeit 0,3 m/s liegen muß. Es hat sich gezeigt, daß man bei Verwendung einer Polymerfolie mit einer Dicke zwischen 5 und 35 µm sehr gute Ergebnisse erzielt. Ein optimales Ergebnis erhält man im allgemeinen mit einer Polymerfoliendicke zwischen 10 und 15 µm.

Die Herstellung von Folien aus den genannten Copolyetherestern erfolgt nach dem an sich bekannten Verfahren, wie es in Kirk-Othmer, Encyclopedia of Chemical Technology 9 (1966), S. 232 - 241, beschrieben ist.

Durch Blasextrusion lassen sich Folien mit einer Dicke zwischen 5 und 35 µm herstellen.

Bevorzugt werden Flachfolien, die man durch Flachextrusion auf eine Kühlwalze erhält. Dabei beträgt die Walzentemperatur vorzugsweise 40 - 120° C, wie in der US-Patentschrift 3 968 183 beschrieben.

Die nach den hierfür beschriebenen Verfahren hergestellten Folien besitzen im allgemeinen ein geringeres Wasseraufnahmevermögen als auf andere Weise, z.B. durch Pressen, erhaltene Folien.

Die Bestimmung der Wasseraufnahme erfolgte an den Schnitzeln. Dabei ging man wie folgt vor: Die Copolyetherester-Schnitzel mit einer Länge von ca. 3 mm und einem Durchmesser von 2,5 mm wurden eine Woche lang in Wasser mit einer Temperatur von 23° C gelagert. Nach sorgfältiger Entfernung der anhaftenden Feuchtigkeit durch Filterpapier wurde die aufgenommene Wassermenge durch Wiegen ermittelt.

Bei der Herstellung von absorptionsfähigen Hygieneprodukten gemäß der vorliegenden Erfindung erhält man die guten Ergebnisse mit Copolyetheresterfolien, die durch Flach-und/oder Blasextrusion aus Polymerschnitzeln hergestellt wurden, die bei Wasserlagerung bei 23° C eine Wasseraufnahme von höchstens 15 Gew.%, bezogen auf das Trockengewicht der Schnitzel, aufweisen.

Dabei werden Polmere bevorzugt, bei denen die daraus hergestellten Schnitzel höchstens 12 Gew.% Wasser aufnehmen. Als flüssigkeitsaufnehmende Sauglage kann man bei den erfindungsgemäßen Hygieneprodukten die von Hygieneprodukten bekannten Absorptionsmaterialien verwenden.
Eine solche Sauglage besteht in vielen Fällen aus einem in Vliesstoff eingelegten Kraftzellstoff.

Wesentlich für gute Eigenschaften und hohen Tragekomfort eines absorptionsfähigen Hygieneproduktes sind neben hoher Wasserdichtheit der wasserundurchlässigen Folie die Absorptionseigenschaften der Sauglage. Diese werden durch die Flüssigkeitsaufnahme, die Flüssigkeitsrückhaltung und die Rückfeuchtung charakterisiert.

Die Messung der Flüssigkeitsaufnahme wird nach der DIN 53 923 durchgeführt.
Als Testflüssigkeiten werden dazu synthetischer Urin oder synthetisches Blut eingesetzt. Synthetischer Urin hat die Zusammensetzung:

| | |
|---|---|
| NaCl | 10 g |
| Harnstoff | 24 g |
| $MgSo_4$ | 0,6 g |
| Ca-acelat | 0,7 g |
| Dest. Wasser | 964,7 g |

Die Oberflächenspannung eines solchen synthetischen Urins beträgt ca. 48 mN/m.
Synthetisches Blut für die Prüfung der Sauglage hatte die Zusammensetzung:

| | |
|---|---|
| NaCl | 10 g |
| Na-carbonat | 10,7 g |
| Glycerin | 100,0 g |
| Carboxymethylcellulose | 4,6 g |
| Dest. Wasser | 874.7 g |

Die Oberflächenspannung beträgt ca. 46 mN/m und die Viskosität ca. 10 mPa • sec.

Der Demand Absorptionstest (Demand Wettability Test) wird den Anforderungen an praxisnahe Prüfung besonders gut gerecht. Dabei wird ein Gerät verwendet, mit dem sich das Volumen und die Geschwindigkeit der Aufnahme einer Flüssigkeit dadurch messen lassen, daß man das absorbierende Material auf einen hydrostatischen Druck von Null einstellt, so daß eine Befeuchtung nur nach Bedarf des absorbierenden Materials stattfindet. Eine Flüssigkeitsaufnahme erfolgt nur aufgrund der Fähigkeit des absorbierenden Materials, Flüssigkeit aufzusaugen, wobei der Flüssigkeitsstrom bei Erreichen des Sättigungspunktes sofort zum Stillstand kommt. Man kann dabei die Fasermasse so aufbereiten, daß das Endprodukt praxisnah simuliert wird (vgl. "Nonwoven Product Technology", International Nonwovens & Disposables Association, New York, 1974, 129 - 142).

Die Messung der Rückfeuchtung (WET-Beck Test) erfolgt in folgender Weise:
0,8 g des absorbierenden Materials werden gleichmäßig über eine Fläche von 100 $cm^2$ (10 cm x 10 cm) verteilt und mit 16 ml einer 1% Kochsalzlösung übergossen. Nach 3 Minuten wird die Fläche mit Papierfilterschichten der Abmessung 11 cm x 11 cm (ca. 20 g) bedeckt und die Fläche mit 35 g/g belastet. Die nach 3 Minuten ermittelte Flüssigkeitsaufnahme im Filterpapier entspricht der Rückgewinnung.

Vorzugsweise werden, um einen hohen Tragekomfort zu gewährleisten, solche Sauglagen eingesetzt, bei denen die Sauglage eine Flüssigkeitsaufnahme von von wenigstens 5 g/g, gemessen nach DIN 53923, ein Rückhaltevermögen nach dem Demand Absorptions-Test von wenigstens 3 g/g und eine Rückfeuchtung höchstens 10% der aufgenommenen Flüssigkeit aufweist.

Die charakteristischen für die erfindungsgemäßen absorptionsfähigen Hygieneprodukte besonders günstigen Eigenschaften der Sauglage, die zusammen mit der Folie aus dem Copolyester hohen Qualitätsanforderungen genügt, werden vorzugsweise dadurch erreicht, daß die Sauglage Polymere mit einem sehr hohen Wasseraufnahmevermögen enthält. Geeignete Produkte sind beispielsweise aus der DE 26 34 994-C2, der DE 27 50 900-A1 und der DE 27 51 822-A1 bekannt.

Im allgemeinen handelt es sich um modifizierte Cellulose und/oder Salze von vernetzten Polycrylaten.

Neben den obengenannten wesentlichen Elementen können die erfindungsgemäßen Hygieneprodukte noch druckempfindliche Bändchen enthalten, die zur Fixierung der Sauglage dienen. Zur Erhöhung des Tragekomforts sowie zur Verhinderung des Austretens von Flüssigkeit kann man auch Bändchen aus einem dehnbaren Material verwenden.

Zur Bestimmung der Eigenschaften der für die erfindungsgemäßen Hygieneprodukte bestimmten Copolyetherester-Folien verwendet man folgende Verfahren:

A) Bestimmung der Wasserdichtheit WD

Hierzu wird eine kreisförmige Probe der zu untersuchenden Folie mit einem Durchmesser von 6,15 cm zwischen eine wasseraufnehmende Schicht und ein Filterpapier mit den gleichen Abmessungen gelegt. Als wasseraufnehmende Schicht verwendet man I g eines Polymers mit einem sehr hohen Wasseraufnahmevermögen, das mit 5 g Wasser gesättigt wurde und sich zwischen zwei Papiertüchern befindet. Das sich nicht an der Folienseite befindende Papiertuch ist mit einer Lage eines hydrophoben Vliesstoffes abgedeckt.

Die Gewichtszunahme des Filterpapiers nach 5 Minuten unter Einwirkung eines Gewichtes von 2 kg, das an der Seite des Vliesstoffes gleichmäßig über das zu untersuchende Produkt verteilt ist, ist ein Maß für die Wasserdichtheit.

B) Bestimmung der Wasserdampfdurchlässigkeit

In eine Zelle mit einem Durchmesser von 5,6 cm werden 3 g eines Polymers mit einem sehr hohen Wasseraufnahmevermögen ("Superslurper"), das mit 20 g Wasser gesättigt wurde, eingebracht. Die zu untersuchende Folie wird dann so über das Polymer gespannt, daß sie durch dieses gut angefeuchtet wird. Die Gewichtsabnahme pro Stunde bei einer Temperatur von 23 °C, einer relativen Luftfeuchtigkeit von 50 % und einer Windgeschwindigkeit von 0,3 m/s ist ein Maß für die Wasserdampfdurchlässigkeit.

C) Die Wasseraufnahme der Polymerschnitzel wird bei 23 °C bestimmt.

<u>Beispiel I</u>

In einen Autoklaven mit einer Kapazität von 200 l wurden 37,6 kg Dimethylterephthalat, 24,4 kg 1,4-Butandiol und 7,5 kg Polyethylenoxidglycol mit einem mittleren Molekulargewicht von 4000 eingebracht. Das Reaktionsgemisch wurde unter Rühren auf 110 °C erhitzt, und danach wurden 500 ppm Tetrabutyltitanat (bezogen auf Dimethylterephthalat) zugefügt. Durch eine weitere Erhöhung der Temperatur auf 160 °C wurde Methanol abdestilliert, wonach der Druck langsam auf 100 Pa reduziert und die Temperatur auf 245 °C erhöht wurde. Während dieser 3 bis 4 Stunden dauernden Polykondensationsreaktion entstand ein Produkt mit einer relativen Viskosität von 2,14 (gemessen bei einer Konzentration von 1,0 g in 100 g m-Cresol bei 25 °C).

In der gleichen Weise wie oben beschrieben erhielt man eine Reihe von Copolyetherestern mit unterschiedlichen Mengen des hierfür angegebenen Polyethylenoxidglycols (PEG) und eventuel Polytetrahydrofuran (PTHF) mit einem mittleren Molekulargewicht von 1000.

Die hergestellten Copolyetherester hatten folgende Zusammensetzung:

Tabelle 1

| Gew.% Estergruppen mit kurzen Ketten | | Gewichtsanteil % | | $\eta$ rel nach Polykondensat. | $\eta$ rel nach Nachkondensat. | Wasseraufn.g/g |
|---|---|---|---|---|---|---|
| | | PEG | PTHF | | | |
| A | 84,5 | 15 | 0 | 2,14 | 3,48 | 6,2 |
| B | 78,8 | 15 | 5 | 2,24 | 3,91 | 9,1 |
| C | 67,5 | 15 | 15 | 2,46 | 4,56 | 11,1 |
| D | 79,3 | 20 | 0 | 2,21 | 4,02 | 10,2 |
| E | 73,7 | 20 | 5 | 2,32 | 4,19 | 12,2 |
| F | 68,0 | 20 | 10 | 2,43 | 4,43 | 14,7 |
| G | 69 | 31 | 0 | - | - | 17,1 |

<u>Beispiel II</u>

Die gemäß Beispiel I hergestellten Copolyetherester wurden zu Folien mit einer Dicke von 15 µm (Copolyetherester E, F und G auch zu Folien mit einer Dicke von 10 µm) verarbeitet, wonach die Wasserdampfdurchlässigkeit (WDD) und die Wasserdichtheit (WD) bestimmt wurden. Die Messergebnisse sind in der nachfolgenden Tabelle wiedergegeben.

Tabelle 2

| Polymer | Gew.% PBTP | η rel d. Folie | WDD ($g/m^2$/24 Std) bei Foliendicke | | WD ($g/cm^2$/Std) bei Foliendicke | |
|---|---|---|---|---|---|---|
| | | | 10 µm | 15 µm | 10 µm | 15 µm |
| A | 84,5 | 2,67 | - | 650 | - | 0,003 |
| B | 78,8 | 2,81 | - | 890 | - | 0,005 |
| C | 67,5 | 3,09 | - | 1760 | - | 0,006 |
| D | 79,3 | 2,61 | - | 1320 | - | 0,007 |
| E | 73,7 | 2,79 | 2700 | 1990 | 0,014 | 0,011 |
| F | 68,0 | 2,91 | 3200 | 2830 | 0,020 | 0,017 |
| G | 69 | 3,5 | 4100 | 3490 | 0,030 | 0,034 |

Vergleichsmessungen mit einem kommerziellen Produkt auf der Basis von mikroporösem Polyethen ergaben eine Wasserdampfdurchlässigkeit von 1060 $g/m^2$/24 Std. und eine Wasserdichheit von 0,006 $g/cm^2$ Std.

Aus den in der obigen Tabelle enthaltenen Ergebnissen geht hervor, daß man aus den Polymeren A bis F Folien mit einer Wasserdichtheit WD $\leq$ 0,02 $g/cm^2$/Std und einer Wasserdampfdurchlässigkeit WDD > 500 $g/m^2$/24 Std erhält. Sie eignen sich hervorragend für absorbierende Hygieneprodukte.

Polymer G ist ein Polymer, aus dem gemäß der US-Patentschrift 4 493 870 eine Folie zur Herstellung von Regenkleidung oder Zelten hergestellt wurde. Eine derartige Folie eignet sich wegen ihrer relativ hohen Wasserdurchlässigkeit weniger gut für die Anwendung in den erfindungsgemäßen Hygieneprodukten. Bei derartigen Produkten muß eine deutlich dickere Folie verwendet werden, damit die Anforderung an die Dichtheit der Folie erfüllt werden kann, was aber den erwünschten Tragekomfort mindert, abgesehen davon, daß der Materialeinsatz wesentlich höher ist.

## Ansprüche

1. Absorptionsfähiges Hygieneprodukt, bestehend aus einer Folie mit einer Wasserdampfdurchlässigkeit von mindestens 500 $g/m^2$/Tag/ 23° C/100-50 % relative Luftfeuchtigkeit/Windgeschwindigkeit 0,3 m/s, einer flüssigkeitsdurchlässigen Schicht und einer flüssigkeits aufnehmenden Sauglage, die Folie aus einem Copolyetherester hergestellt wurde, der aus einer Vielzahl sich wiederholender intralinearer Estergruppen mit einem langen Kettenmolekül und Estergruppen mit einem kurzen Kettenmolekül besteht, die durch Esterbindungen auf statistische Weise so miteinander verbunden sind, daß der Anfang und das Ende jeweils aneinander angrenzen, wobei die Estergruppen mit langkettigen Estereinheiten der folgenden Formel entsprechen:

$$-O\,G\,O\,\overset{\overset{O}{\|}}{C}\,R\,\overset{\overset{O}{\|}}{C}-$$

und wobei die Estergruppen mit kurzkettigen Estereinheiten lekül der folgenden Formel entsprechen:

$$-O\,G\,O\,\overset{\overset{O}{\|}}{C}\,R\,\overset{\overset{O}{\|}}{C}-$$

wobei G einen zweiwertigen Rest darstellt, die nach Entfernung von endständigen Hydroxylgruppen von mindestens ein Glycol mit einem langen Kettenmolekül, einem Molekulargewicht von mehr als 800 und einem mittleren Atomverhältnis von Kohlenstoff zu Sauerstoff von 2,0 übrigbleibt, wobei R einen zweiwertigen Rest darstellt, der nach Entfernung von Carboxylgruppen von mindestens einer Carbonsäure mit einem Molekulargewicht von weniger als 300 übrigbleibt, und D einen zweiwertigen Rest darstellt, der nach Entfernung von Hydroxylgruppen von mindestens einem Diol mit einem Molekulargewicht von weniger als 250 übrigbleibt, wobei die verwendete Dicarbonsäure zu mindestens 75 Mol% aus Terephthalsäure oder deren esterbildenden Äquivalenten besteht und das Diol mit einem niedrigen Molekulargewicht zu mindestens 75 Mol% aus 1,4-Butandiol oder dessen esterbildenden Äquivalenten besteht, die Summe der Molprozente der Dicarbonsäure, die keine Terephthalsäure oder dessen esterbildendes Äquivalent ist, und des Diols mit einem niedrigen Molekulargewicht, das kein 1,4-Butandiol oder dessen esterbildendes Äquivalent ist, höchstens 25 beträgt, dadurch gekennzeichnet, daß der Copolyetherester zu 45 - 95 Gew.% aus kurzkettigen Estereinheiten besteht, die Flüssigkeitsdurchlässigkeit von maximal 0.02 $g/cm^2$/Std/23°C/0,07 bar und im Copolyetherester der Gewichtsanteil der langkettigen Estereinheiten 5 - 55% beträgt und das mittlere Kohlenstoff/Sauerstoff-Verhältnis der langkettigen Glycole zwischen 2,0 und 3,7 liegt und dessen Molekulargewicht 800 - 6000 beträgt mit der Maßgabe, daß, wenn das mittlere Atomverhältnis Kohlenstoff zu Sauerstoff der langkettigen Glycole zwischen 2,0 und 2,4 liegt, der Gewichtsanteil der langkettigen Estereinheiten 5 - 35% beträgt, und die Copolyetherester-Folie durch Flach- und/oder Blasextrusion aus Polymerschnitzeln, die in Wasser von 23°C eine Wasseraufnahme von höchstens 15 Gew.%, bezogen auf das Trockengewicht der Schnitzel, aufweisen, hergestellt wurden.

2. Absorptionsfähiges Hygieneprodukt nach Anspruch 1, dadurch gekennzeichnet, daß die Sauglage eine Flüssigkeitsaufnahme von wenigstens 5 g/g, gemessen nach DIN 53923, ein Rückhaltevermögen nach dem Demand Absorptions-Test von wenigstens 3 g/g und eine Rückfeuchtung höchstens 10% der aufgenommenen Flüssigkeit aufweist.

3. Absorptionsfähiges Hygieneprodukt nach Anspruch 2, dadurch gekennzeichnet, daß die Sauglage Polymere mit einem sehr hohen Wasseraufnahmevermögen enthält.

4. Absorptionsfähiges Hygieneprodukt gemäß Anspruch 1, dadurch gekennzeichnet, daß es sich bei dem im Copolyetherester vorhandenen langkettigen Glycol mit mit einem Kohlenstoff/Sauerstoff-Verhältnis zwischen 2,0 und 3,7 um ein Polyalkylenoxidglycol mit einem Molekulargewicht zwischen 1000 und 5000 handelt.

5. Absorptionsfähiges Hygieneprodukt gemäß Anspruch 1, dadurch gekennzeichnet, daß im Copolyetherester die kurzkettigen Estereinheiten hauptsächlich aus Polybutylenterephthalat-Gruppen bestehen.

6. Absorptionsfähiges Hygieneprodukt gemäß Anspruch 1, dadurch gekennzeichnet, daß die Dicke der Copolyetherester-Folie zwischen 5 und 35 µm, vorzugsweise zwischen 10 und 20 µm liegt.

7. Absorptionsfähiges Hygieneprodukt gemäß Anspruch 1, dadurch gekennzeichnet, daß die Copolyetherester-Schnitzel eine Wasseraufnahme von höchstens 12 Gew.% aufweisen.

Europäisches Patentamt

# EUROPÄISCHER RECHERCHENBERICHT

Nummer der Anmeldung

EP 88 11 6748

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y,D | US-A-4 493 870 (C. VROUENRAETS)<br>* Insgesamt *<br>--- | 1-7 | A 61 L 15/00<br>C 08 G 63/66 |
| Y,D | EP-A-0 192 965 (KAO)<br>* Anspruch 1 *<br>--- | 1-7 | |
| A,D | DE-A-2 634 994 (AKZO)<br>* Insgesamt *<br>--- | 2-3 | |
| A,D | GB-A-1 403 210 (DU PONT DE NEMOURS)<br>* Insgesamt *<br>--- | 1-7 | |
| A | EP-A-0 158 490 (PERSONAL PRODUCTS CO.)<br>* Seite 12, Zeile 9 - Seite 13, Zeile 24 *<br>----- | 1-7 | |

RECHERCHIERTE SACHGEBIETE (Int. Cl.4)

A 61 L
C 08 G

Der vorliegende Recherchenbericht wurde für alle Patentansprüche erstellt

| Recherchenort | Abschlußdatum der Recherche | Prüfer |
|---|---|---|
| DEN HAAG | 18-01-1989 | COUSINS-VAN STEEN G.I.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)